# EUROPEAN PATENT SPECIFICATION

(11) **EP 2 460 512 B1**
(45) Date of publication and mention of the grant of the patent: **08.11.2017**
(21) Application number: 10015248.7
(22) Date of filing: 03.12.2010
(51) Int. Cl.: A61K 8/81, A61Q 5/04

(54) **Process for permanent shaping of human hair comprising dispersed particles of water insoluble polymer**
Verfahren zur dauerhaften Verformung von menschlichem Haar enthaltend dispergierte Partikeln eines wasserunlöslichen Polymers
Procédé de mise en forme permanente de cheveux humains comprenant une dispersion de particules de polymère insoluble dans l'eau

(43) Date of publication of application: 06.06.2012
(73) Proprietor: Kao Germany GmbH, 64297 Darmstadt (DE)
(72) Inventor: Schmelz, Sandra, 97828 Marktheidenfeld (DE); Meuser, Alexandra, Dr., 63329 Egelsbach (DE)
(74) Representative: Grit, Mustafa

(56) References cited:
- EP-A1- 2 191 865
- JP-B2- 3 115 378
- US-A- 5 163 010
- US-A- 5 441 729
- US-A- 6 080 390
- US-A1- 2003 035 783

## Description

The present invention concerns a process for the permanent shaping of human hair used both for the permanent waving of human hair with an excellent waving effect as well as for the straightening of either naturally or chemically curled hair wherein optimum hair conditioning is observed.

It is generally known that permanent waving is carried out in two steps, the reductive splitting of the cysteine disulfide bonds in the hair by a reducing agent and the subsequent neutralization by application of an oxidizing agent, whereby the cysteine disulfide bonds are restored.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycolates are customarily applied at a pH-value between 7 and 10, in particular 8.5 and 9.5.

Such compositions vary in their waving and/or straightening performance and, therefore, there is still need for further improvement, especially in permanent shaping of damaged to strongly damaged hair and in particular for permanent shaping hair streaks including parts with various damage level in length and also improvement in hair condition after such carrying our permanent shaping process.

US 5.441.729 discloses a method for reducing post permanent wave odors from hair using a salt comprising intermediate treatment composition which is applied onto hair after reducing composition. The compositions disclosed may comprise cationic polymer and anionic styrene-acrylate copolymer.

The present invention starts from the task of providing a process for the permanent shaping of human hair with excellent waving and straightening performance. Hair waved or straightened with the process disclosed herein looks and feels natural upon touching by hand and has improved shine. For waved hair it is especially important that the hair has excellent elasticity and bounce.

Accordingly, the first object of the present invention is a process for permanent shaping hair wherein
- hair is washed or shampooed or made wet and
- applied a composition comprising at least one reducing agent, at least one cationic polymer and dispersed particles of at least one water insoluble non-ionic polymer of styrene-acrylic acid copolymer and
- processed for 1 to 30 min and
- rinsed off from hair and
- subsequently a composition comprising at least one oxidizing agent is applied and
- after processing of 1 to 30 min rinsed off from hair,
wherein a composition comprising at least one inorganic salt is applied onto hair after rinsing off the reducing agent composition and before applying oxidizing agent comprising composition and is not rinsed off.

In the above processes, in case the process is carried out for curling of hair, hair is wound on the curlers either after shampooing or washing or wetting or during the application of the reducing agent or after application of the reducing agent. The curlers are taken off from hair after rinsing off the reducing agent or after application of intermediate treatment or before application of the oxidizing agent or after application of the oxidizing agent or at the end of the processing time of oxidizing agent. The time of wounding onto curlers and taking them off is very much dependent on the curling strength aimed. For stronger curls it is preferred that the wounding onto curlers is done before application of the reducing agent and curlers are taken off at the end of the processing time of oxidizing agent.

Within the meaning of the present invention it is meant with the term "water insoluble polymer" that the polymer is practically insoluble in water at any temperature permanent shaping is carried out, i.e. a temperature up to 50°C.

Suitable particles of water insoluble polymer comprises styrene-acrylic acid copolymer. Polymers suited are of non-ionic ones. Most preferred particles of water insoluble polymer dispersed in water are of styrene - acrylates colpolymer available from Interpolymer under the trade name Syntran 5905.

Concentration of dispersed particles of a water insoluble polymer is in the range of 0.001 to 5%, preferably 0.01 to 3% and more preferably 0.02 to 2% and most preferably 0.05 to 1% by weight calculated to total composition. It should be noted that in case all compositions, i.e. reducing composition and intermediate treatment composition and oxidizing composition comprise the dispersed particles of a water insoluble polymer, the concentration mentioned here is in each composition and calculated to total of each composition.

The reducing composition according to the invention comprises at least one reducing compound at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting therefrom, 1.3-butanediol and 1.4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the composition according to the invention customarily amounts from 0.5 to 15 %, preferably 1.0 to 12.5%, more preferably 2.0 to 12.5% by weight, calculated to total composition as free thioglycolic acid as reference substance.

The compositions containing reducing agents can, if necessary, comprise alkalizing agents. Their quantity is dependent on the reducing agent and the desired pH-value of the composition. Reducing agent compositions preferably contain 0.1 % to 5%, in particular 0.5% to 2.5% by weight thereof, calculated to the total composition. Alkalizing agents preferred within the scope of the invention are ammonium carbamate, ammonia and/or ammonium(bi)carbonate, monoethanolamine and triethanolamine. It is desirable to adjust the pH-value between about 6.5 and 10.5, preferably about 7 to 9.5.

The process according to the invention is suited for use both for the permanent waving, i.e. curling of human hair and for the straightening, i.e. smoothing thereof.

The viscosity best suited for the reducingcomposition according to the process of the present invention proved to be in the range of 1 to 10,000 mPa.s, preferably about 1 to about 5,000 mPa.s, measured at 20°C in a Brookfield viscosimeter (no. 5 spindle), whereas the viscosity suited for the straightening compositions is preferably higher in a range up to 50,000 mPa.s, preferably up to 30,000 mPa.s measured at 20°C in a Brookfield viscosimeter (no. 5 spindle).

The viscosity is adjusted by addition of the appropriate amounts of thickening agents known **per se,** such as cellulose derivatives. Thickening may as well be realized by formulating a composition in form of an emulsion with the use of C₁₀-C₂₂-fatty alcohols, in mixture with long mono alkyl chain quaternary ammonium surfactants.

The reducing composition according to the present invention preferably comprises surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, permanent shaping compositions comprise at least one cationic surfactant according to general formula 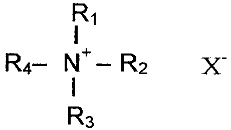
where R₁s a saturated or unsaturated, branched or non-branched alkyl chain with 8-24 C atoms or

   R₅CO NH (CH₂)ₙ
where R₅ is saturated or unsaturated, branched or non-branched alkyl chain with 7-21 C atoms and n has typical value of 0 - 4 or

   R₆CO O (CH₂)ₙ
where R₆ is saturated or unsaturated, branched or non-branched alkyl chain with 7-21 C atoms and n has typical value of 0 - 4, and
R₂ is a saturated or unsaturated, branched or non-branched alkyl chain with 1-24 C atoms or

   R₅CO NH (CH₂)ₙ
where R₅ is saturated or unsaturated, branched or non-branched alkyl chain with 7-21 C atoms and n has typical value of 0 - 4 or

   R₆ CO O (CH₂)ₙ
where R₆ is saturated or unsaturated, branched or non-branched alkyl chain with 7-21 C atoms and n has typical value of 0 - 4,
and R₃ and R₄ are same or different lower alkyl chain with 1 to 4 carbon atoms which may be substituted with one or two hydroxyl group, and X is chloride, bromide or methosulfate.

Concantration of cationic surfactant is in the range from 0.05 % to 5 %, preferably 0.1 % to 2.5 % by weight, calculated to total composition.

Suitable long-chain quaternary ammonium compounds are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

Further the reducing composition used in the process of the present invention comprises cationic polymer. Basically suitable are all cationic polymers listed under the generic name "Polyquaternium" in the CTFA International Cosmetic Ingredient Dictionary. Examples are Polyquaternium 4, Polyquaternium 5, Polyquaternium 6, Polyquaternium 7, Polyquaternium 10, Polyquaternium 11, Polyquaternium 16, Polyquaternium 22 and Polyquaternium 28, Polyquaternium 2, Polyquaternium 87, and Polyquaternium 39,

The cationic polymers also include the quaternized products of graft polymers from organopolysiloxanes and polyethyl oxazolines described in EP-A 524 612 and EP-A 640 643. Such polymer is known with its CTFA name Polysilicone-9.

Concantration of cationic polymers is in the range from 0.05 % to 2.5 %, preferably 0.1 % to 1.5 % by weight, calculated to total composition.

The reducing compositions of present invention can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

The reducing composition can comprise further ceramide type of compound such as cetyl-PG-hydroxyethylpalmitamide.

Further optional ingredient are sterols,especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 2.5% by weight calculated to total composition.

Another preferred compound in the reducing composition is silicone compounds and especially aminated silicones such as amodimethicone available from for example Dow Corning under the brand names Dow Corning 949 Emulsion and Dow Corning 2-8194. Concentration of silicones, especially amodimethicone, is in the range of 0.05 to 2.5%, preferably 0.1 to 1% by weight calculated to total composition.

Additionally, one or more natural oil component may be incorporated into the redcuing composition. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further additional compounds may be present in the reducing compositions is ubichinone of the formula 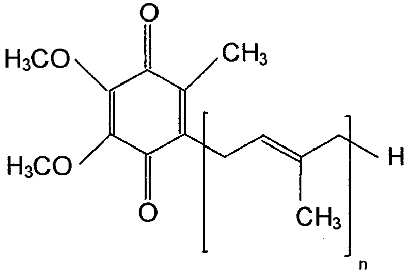 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in permanent shaping compositions of the present invention is from 0.0001 to 1%, preferably from 0.0002 to 0.75%, more preferably from 0.0002 to 0.5% and most preferably from 0.0005 to 0.5% by weight, calculated to total composition.

In another preferred embodiment of the present invention, the reducing composition comprises one or more fatty alcohol with a chain length of 12 to 24 C atoms. Concentration of one or more fatty alcohol is in the range of 1 to 15%, preferably 1 to 10%, more preferably 2 to 7.5 and most preferably 2 to 5% by weight calculated to total composition.

Suitable non limiting examples are lauryl alchohol, myristyl alcohol, cetyl alcohol, stearyl alcohol cetearyl alcohol, behenyl alcohol and their mixtures. Preferred are cetyl alcohol, stearyl alcohol cetearyl alcohol, behenyl alcohol and their mixtures. Particularly preferred are cetyl alcohol, stearyl alcohol and their 1 to 1, by weight, mixture cetearyl alcohol.

The reducing composition used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Öle-Fette-Wachse", 117 (1991), pages 81 to 87.

The oxidizing composition may also comprise one or more of the above mentioned ingredients. In any case oxidizing composition comprises at least one oxidizing agent preferably selected from hydrogen peroxide or sodium bromate and preferably at a concentration of 0.5 to 10% by weight calculated to total composition. Oxidizing composition has a pH between 2 and 5, preferably between 2 and 4.

The intermediate treatment composition comprises at least one inorganic salt and has a pH value between 2.5 to 6, preferably 3 to 5.5 and most preferably 3 to 5. The inorganic salt concentration may very between 1 and 20% and preferably 2 and 15% and more preferably 5 and 15% by weight calculated to total of the composition.

A straightening process may also be carried out in a different process wherein hair is washed and/or shampooed and dried and reducing composition is applied onto hair and processed for 5 to 45 min and rinsed off from hair and hair is dried, the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and optionally rinsed off, wherein at least one of the two composition comprises dispersed particles of at least one water insoluble polymer.

In a further straightening process after physically straightening hair with hot iron at a temperature of 130 to 210°C and prior to application of the oxidizing agent an intermediate treatment composition comprising at least one inorganic salt may be applied onto hair and without rinsing off hair is treated as disclosed above.

The compositions using in permanent shaping process is offered to the users preferably in the form of a kit. Accordingly further object of the present invention is a kit for permanent shaping hair which comprises at least two compositions kept separately wherein one of the compositions comprises at least one reducing agent at least one cationic polymer and dispersed particles of at least one water insoluble non-ionic polymer of styrene-acrylic acid copolymer and the other comprises at least one oxidizing agent preferably selected from sodium bromate and hydrogen peroxide.

The following examples are to illustrate the invention, but not to limit it.

### Example 1: - (Not within the scope of the claims)

| | |
|---|---|
| Ammonium thioglycolate (60%) | 21.3 (% by wt. |
| Ammonium hydrogen carbonate | 5.0 |
| 1,3- butylene gylcol | 3.0 |
| Syntran 5905 | 1.0 |
| PEG-40-Hydrogenated castor oil | 0.7 |
| Perfume | 0.4 |
| Ammonia, 25% | ad pH 8.3 |
| Water | ad 100.0 |

With this composition the hair was permanently waved for about 15 minutes, rinsed and neutralized for about 8 minutes with a customary 2.5% H₂O₂ composition. Homogeneous wave appearance was obtained which had excellent elasticity and bounce and improved shine and felt natural and soft upon touching. Exclusion of Syntran 5905 resulted in less homogeneous perm appearance.

Additionally Syntran 5905 was removed from the reducing composition and was added at the same concentration into the oxidizing composition and hair was permed with a reducing composition without Syntran 5905 and an oxidizing composition with Syntran 5905. Here again Homogeneous wave appearance was obtained which had excellent elasticity and bounce and improved shine and felt natural and soft upon touching. It should be noted the results were slightly better and preferred compared to the results obtained when Syntran 5905 was comprised in reducing composition.

In a third trial, Syntryn 5905 was used in both reducing and oxiding agnet whereas the concentrations were halved, i.e. in each composition 0.5% Syntran 5905 was used. Homogeneous wave appearance was obtained which had excellent elasticity and bounce and improved shine and felt natural and soft upon touching. When compared the perming efficiency, results obtained with this trial were superior to the previous results described above.

In the above process the use of intermediate treatment according to following composition improved the perming result further.

### Intermediate treatment composition

| | |
|---|---|
| Asparagic acid | 0.25% by weight |
| Glutamic acid | 0.50 |
| Alanin DL | 0.25 |
| Magnesium sulfate | 10.00 |
| Water | q.s. to 100 |

The above composition had a pH of 4.10.

### Example 2: (Not within the scope of the claims)

### Straightening Composition

| Thioglycolic acid | 8.0 (% by wt.) |
|---|---|
| C₁₆-C₂₂-Fatty alcohol mixture | 3.5 |
| Oleth-50 | 2.5 |
| Laureth-23 | 1.5 |
| Syntran 5905 | 2.0 |
| Ethanol | 5.0 |
| Perfume | 0.6 |
| Monoethanolamine | ad pH 9.3 |
| Water | ad 100.0 |

This composition constitutes an effecting smoothing composition for kinky hair.

## Claims

1. Process for permanent shaping hair **characterized in that**
- hair is washed or shampooed or made wet and
- applied a composition comprising at least one reducing agent, at least one cationic polymer and dispersed particles of at least one water insoluble non-ionic polymer of styrene-acrylic acid copolymer and
- processed for 1 to 30 min and
- rinsed off from hair and
- subsequently a composition comprising at least one oxidizing agent is applied and
- after processing of 1 to 30 min rinsed off from hair,
wherein a composition comprising at least one inorganic salt is applied onto hair after rinsing off the reducing agent composition and before applying oxidizing agent comprising composition and is not rinsed off.

2. The process according to claim 1 **characterized in that** the reducing agents are selected from thioglycolic acid, thiolactic acid and their salts, cystein and its hydrochloride salt, acetylcystein.

3. The process according to claims 1 and 2 **characterized in that** the reducing agents are comprised at a concentration of 0.5 to 15% by weight, calculated to total composition.

4. The process according to any of the preceding claims **characterized in that** at least one water insoluble polymer is comprised at a concentration in the range of 0.001 to 5% by weight calculated to total composition.

5. The process according to any of the preceding claims **characterized in that** the reducing composition comprises at least one surfactant selected from anionic, nonionic, cationic and amphoteric ones.

6. The process according to claim 5 **characterized in that** at least one surfactant is comprised at a concentration of 0.05 to 10% by weight, calculated to total composition.

7. The process according to any of the preceding claims **characterized in that** the composition comprising at least one inorganic salt comprises inorganic salt at a concentration in the range between 1 and 20% by weight calculated to the total composition.

8. The process according to any of the preceding claims **characterized in that** the composition comprising at least one inorganic salt has a pH in the range between 2.5 and 6.

9. The process according to any of the preceding claims **characterized in that** the reducing composition has a pH in the range of 6.5 and 10.5.

10. The process according to any of the preceding claims **characterized in that** the reducing composition comprises an alkalizing agent.

## Patentansprüche

1. Verfahren zum dauerhaften Formen von Haaren, **dadurch gekennzeichnet, dass**
- die Haare gewaschen oder shampooniert oder nass gemacht werden und
- eine Zusammensetzung aufgebracht wird, welche mindestens ein Reduktionsmittel, mindestens ein kationisches Polymer und dispergierte Teilchen mindestens eines wasserunlöslichen nichtionischen Polymers eines Styrol-Acrylsäure-Copolymers aufweist, und
- man sie 1 min bis 30 min lang einwirken lässt und
- sie aus den Haaren ausgespült wird und
- anschließend eine Zusammensetzung aufgebracht wird, welche mindestens ein Oxidationsmittel aufweist, und
- sie nach einem Einwirken von 1 min bis 30 min aus den Haaren ausgespült wird,
wobei nach dem Ausspülen der Reduktionsmittelzusammensetzung und vor dem Aufbringen der Zusammensetzung, die ein Oxidationsmittel aufweist, eine Zusammensetzung, welche mindestens ein anorganisches Salz aufweist, auf die Haare aufgebracht wird und nicht ausgespült wird.

2. Verfahren nach Anspruch 1, **dadurch gekennzeichnet, dass** die Reduktionsmittel aus Thioglykolsäure, Thiomilchsäure und deren Salzen, Cystein und dessen Hydrochloridsalz, Acetylcystein ausgewählt sind.

3. Verfahren nach Anspruch 1 und 2, **dadurch gekennzeichnet, dass** die Reduktionsmittel in einer Konzentration von 0,5 Gewichts-% bis 15 Gewichts-% enthalten sind, bezogen auf die Gesamtzusammensetzung.

4. Verfahren nach einem der vorhergehenden Ansprüche, **dadurch gekennzeichnet, dass** mindestens ein wasserunlösliches Polymer in einer Konzentration im Bereich von 0,001 Gewichts-% bis 5 Gewichts-% enthalten ist, bezogen auf die Gesamtzusammensetzung.

5. Verfahren nach einem der vorhergehenden Ansprüche, **dadurch gekennzeichnet, dass** die reduzierende Zusammensetzung mindestens ein Tensid aufweist, das aus anionischen, nichtionischen, kationischen und amphoteren ausgewählt ist.

6. Verfahren nach Anspruch 5, **dadurch gekennzeichnet, dass** mindestens ein Tensid in einer Konzentration von 0,05 Gewichts-% bis 10 Gewichts-% enthalten ist, bezogen auf die Gesamtzusammensetzung.

7. Verfahren nach einem der vorhergehenden Ansprüche, **dadurch gekennzeichnet, dass** die Zusammensetzung, die mindestens ein anorganisches Salz aufweist, anorganisches Salz in einer Konzentration im Bereich von 1 Gewichts-% bis 20 Gewichts-% aufweist, bezogen auf die Gesamtzusammensetzung.

8. Verfahren nach einem der vorhergehenden Ansprüche, **dadurch gekennzeichnet, dass** die Zusammensetzung, die mindestens ein anorganisches Salz aufweist, einen pH-Wert im Bereich von 2,5 bis 6 aufweist.

9. Verfahren nach einem der vorhergehenden Ansprüche, **dadurch gekennzeichnet, dass** die reduzierende Zusammensetzung einen pH-Wert im Bereich von 6,5 bis 10,5 aufweist.

10. Verfahren nach einem der vorhergehenden Ansprüche, **dadurch gekennzeichnet, dass** die reduzierende Zusammensetzung ein Alkalisierungsmittel aufweist.

## Revendications

1. Procédé de mise en forme permanente des cheveux **caractérisé en ce que**
- les cheveux sont lavés, ou shampouinés, ou mouillés, et
- une composition comprenant au moins un agent réducteur, au moins un polymère cationique et des particules dispersées à base d'au moins un polymère non ionique insoluble dans l'eau d'un copolymère styrène-acide acrylique, est appliquée, et
- laissée pour un traitement pendant 1 à 30 minutes, et
- est enlevée des cheveux par rinçage, et
- ensuite une composition comprenant au moins un agent oxydant est appliqué, et
- après un traitement de 1 à 30 minutes est enlevé des cheveux par rinçage,
où une composition comprenant au moins un sel inorganique est appliquée sur les cheveux après le rinçage de la composition d'agent réducteur et avant l'application de la composition comprenant l'agent oxydant et n'est pas enlevée par rinçage.

2. Procédé selon la revendication 1, **caractérisé en ce que** les agents réducteurs sont choisis parmi l'acide thioglycolique, l'acide thiolactique et leurs sels, la cystéine et son sel chlorhydrate, l'acétylcystéine.

3. Procédé selon les revendications 1 et 2, **caractérisé en ce que** les agents réducteurs sont compris à une concentration de 0,5 à 15 % en poids, calculée par rapport à la composition totale.

4. Procédé selon l'une quelconque des revendications précédentes, **caractérisé en ce qu'**au moins un polymère insoluble dans l'eau est compris à une concentration dans la plage de 0,001 à 5 % en poids, calculée par rapport à la composition totale.

5. Procédé selon l'une quelconque des revendications précédentes, **caractérisé en ce que** la composition réductrice comprend au moins un tensioactif choisi parmi les tensioactifs anioniques non ioniques, cationiques et amphotères.

6. Procédé selon la revendication 5, **caractérisé en ce qu'**au moins un tensioactif est compris à une concentration de 0,05 à 10 % en poids, calculée par rapport à la composition totale.

7. Procédé selon l'une quelconque des revendications précédentes, **caractérisé en ce que** la composition comprenant au moins un sel inorganique comprend un sel inorganique à une concentration dans la plage entre 1 et 20 % en poids, calculée par rapport à la composition totale.

8. Procédé selon l'une quelconque des revendications précédentes, **caractérisé en ce que** la composition comprenant au moins un sel inorganique a un pH dans la gamme entre 2,5 et 6.

9. Procédé selon l'une quelconque des revendications précédentes, **caractérisé en ce que** la composition réductrice a un pH dans la gamme de 6,5 et 10,5.

10. Procédé selon l'une quelconque des revendications précédentes, **caractérisé en ce que** la composition réductrice comprend un agent d'alcalisation.
